# EUROPÄISCHE PATENTSCHRIFT

(11) **EP 0 832 982 B1**
(45) Veröffentlichungstag und Bekanntmachung des Hinweises auf die Patenterteilung: **15.05.2002**
(21) Anmeldenummer: 97115612.0
(22) Anmeldetag: 09.09.1997
(51) Int. Cl.: C12P 13/20, C07K 5/06, C07C 271/22

(54) **Verfahren zur Herstellung von Z-L-Asparaginsäure-Dinatriumsalz aus Fumarsäure**
Process for the preparation of the disodium salt of z-l-aspartic acid from fumaric acid
Procédé de préparation du sel disodique de l'acide z-l-aspartique à partir de l'acide fumarique

(30) Priorität: 20.09.1996 AT 167296; 19.02.1997 AT 27297
(43) Veröffentlichungstag der Anmeldung: 01.04.1998
(73) Patentinhaber: DSM Fine Chemicals Austria Nfg GmbH & Co KG, 4021 Linz (AT); Holland Sweetener Company V.o.F., 6212 XW Maastricht (NL)
(72) Erfinder: Kirchner, Gerald, 46485 Wesel (DE); Salzbrenner, Erik, 4111 Walding (AT); Werenka, Christian, 4052 Ansfelden (AT); Boesten, Wilhelmus, 6132 BJ Sittard (NL)
(74) Vertreter: Klostermann, Ingrid

(56) Entgegenhaltungen:
- EP-A- 0 612 784
- EP-A- 0 613 920
- DE-A- 3 029 348
- DE-A- 3 123 668
- FR-A- 2 158 330
- US-A- 3 214 345
- PATENT ABSTRACTS OF JAPAN vol. 096, no. 003, 29.März 1996 & JP 07 308195 A (NIPPON SHOKUBAI CO LTD), 28.November 1995,

## Beschreibung

L-Asparaginsäure ist ein essentieller Ausgangsstoff für die Synthese des Dipeptides Aspartam, ein künstlicher Süßstoff mit einer +/- 200-fachen Süßkraft von Saccharose.

Für die Synthese von Aspartam wurden bereits eine Vielzahl von chemischen und enzymatischen Verfahren beschrieben, bei welchen vor allem L-Asparaginsäure und Phenylalaninmethylester auf unterschiedlichste Weisen, etwa mit oder ohne enzymatischer Katalyse, miteinander gekoppelt werden. Dabei wird beispielsweise von fester Asparaginsäure ausgegangen, die nach Umwandlung in das Dinatriumsalz mit Benzyloxycarbonylchlorid (Z-CI) in wäßriger Lösung zu Z-L-Asparaginsäure-Dinatriumsalz umgesetzt wird DE A 3 123 668 Asparaginsäure wird dabei, wie beispielsweise in EP-A-0 127 940 beschrieben, aus Maleinsäure, die zur Fumarsäure isomerisiert wird, auf enzymatischem Wege über das Ammonium-L-Aspartat und anschließender Kristallisation in Gegenwart einer Säure hergestellt. Dabei fallen in der Mutterlauge äquimolare Mengen an Ammoniumsalzen an, die entsprechend entsorgt werden müssen. Weiters ist eine Vielzahl von Schritten erforderlich, um Z-L-Asparaginsäure-Dinatriumsalz zu erhalten.

Aufgabe der vorliegenden Erfindung war es daher, ein Verfahren zu finden, bei welchem die Ammoniumionen-hältigen Abwasser vermieden und die Anzahl der Schritte zum Z-L-Asparaginsäure-Dinatriumsalz reduziert wird.

Unerwarteterweise konnte diese Aufgabe durch ein Verfahren gelöst werden, bei welchem, ausgehend von Fumarsäure, Ammonium-L-Aspartat direkt in das Dinatriumsalz der L-Asparaginsäure bei gleichzeitiger quantitativer Rückführung des Ammoniaks überführt wird.

Gegenstand der vorliegenden Erfindung ist demnach ein Verfahren zur Herstellung von Z-L-Asparaginsäure-Dinatriumsalz aus Fumarsäure, das dadurch gekennzeichnet ist, daß
a) Fumarsäure in einem inerten Verdünnungsmittel in Gegenwart von Aspartase oder Aspartase-produzierenden Mikroorganismen mit Ammoniak zu Ammonium-L-Aspartat umgesetzt, anschließend
b) die Ammonium-L-Aspartat hältige Lösung mit Natriumhydroxid versetzt wird, wobei sich je nach eingesetzter Menge an Natriumhydroxid das Mono- oder Dinatrium-L-Aspartat oder ein Gemisch davon bildet und
c) der sich abspaltende Ammoniak in eine Fumarsäuresuspension, die als Ausgangslösung für weitere enzymatische Umsetzungen verwendet wird, rückgeführt wird, worauf
d) die verbleibende Mono- und/oder Dinatrium-L-Aspartat hältige Lösung mit Benzyloxycarbonylchlorid bei einem pH-Wert zwischen 9 und 14 bei gleichzeitiger Zugabe von Natriumhydroxid reagieren gelassen wird, sodaß sich Z-L-Asparaginsäure-Dinatriumsalz bildet.

Im ersten Schritt a) des erfindungsgemäßen Verfahrens wird Fumarsäure in einer enzymatischen Reaktion zu Ammonium-L-Aspartat umgesetzt.
Das Ausgangsprodukt für das erfindungsgemäße Verfahren, die Fumarsäure kann beispielsweise durch Isomerisierung von Maleinsäure hergestellt werden.

Für die enzymatische Reaktion wird Fumarsäure bis zur Löslichkeitsgrenze in ein inertes Verdünnungsmittel eingerührt, sodaß eine Suspension erhalten wird. Als inerte Verdünnungsmittel eignen sich Wasser, Wasser/Ethanol- oder Wasser/Aceton Gemische und dergleichen.
Bevorzugt wird Wasser eingesetzt. In die Fumarsäure-Suspension wird sodann bei Raumtemperatur Ammoniak verflüssigt oder in Form einer 10 bis 35 Gew.%igen Lösung eingeleitet, wodurch sich die Temperatur bis zu 60°C erhöht und ein pH-Wert zwischen 8 und 9 einstellt.
Bevorzugt wird eine wäßrige 20 bis 30 Gew.%ige Ammoniaklösung verwendet. In das so erhaltene System, vorzugsweise eine Lösung, wird sodann bei 20 bis 60°C, bevorzugt bei 30 bis 50°C das Enzym Aspartase oder ein Aspartaseproduzierender Mikroorganismus, eingerührt. Bei dieser Zugabe an Enzym- bzw. Aspartase-produzierenden Mikroorganismus ist es von Vorteil, wenn durch die Ammoniakzugabe eine Lösung erhalten wird, da im Falle einer Suspension durch Adsorption des Enzyms und dadurch bedingten Aktivitätsverlust mehr Enzym erforderlich ist. Für einen fast quantitativen Umsatz nach bis zu 24 bis 30 Stunden sind dabei 30 bis 50 IU (Enzymaktivität) pro Mol Fumarsäure erforderlich.
Aspartase-produzierende Mikroorganismen sind beispielsweise Pseudomonas fluorescens, Proteus vulgaris, Pseudomonas aeruginosa, Serratia marcescens, Bacterium succinium, Bacillus subtilis, Aerobacter aerogenes, Micrococcus sp. Escherichia coli u. a.
Weitere geeignete Aspartase-produzierende Mikroorganismen sind beispielsweise in US 3,791,926 und US 3,198,712 beschrieben.
Bei dem erfindungsgemäßen Verfahren kann weiters gereinigte oder synthetische Aspartase eingesetzt werden. Das Enzym bzw. der Aspartase-produzierende Mikroorganismus kann in flüssiger oder in immobilisierter Form wie beispielsweise in EP 0 127 940 beschrieben, zugesetzt werden.
Nach vollendeter Reaktion, das Reaktionsende kann beispielsweise photometrisch ermittelt werden, wird die das Ammonium-L-Aspartat enthaltende Lösung bevorzugt auf 20 bis 30°C abgekühlt und im 2. Schritt (b) mit Natriumhydroxid versetzt. Die Menge an zugesetztem Natriumhydroxid ist dabei abhängig vom gewünschten Natriumsalz. Für die Weiterreaktion eignen sich dabei sowohl das Mononatrium- und das Dinatriumsalz als auch Gemische davon. Natriumhydroxid kann dabei in fester Form, oder als Natronlauge eingesetzt werden. Bevorzugt wird Natronlauge eingesetzt. Die Reaktionslösung erwärmt sich dabei auf etwa 40 bis 60°C. Der sich abspaltende und entweichende Ammoniak wird beispielsweise in einem weiteren Reaktionsgefäß mittels Tauchrohr in eine Fumarsäuresuspension, die nach weiterer Zugabe von Ammoniak wieder als Ausgangslösung für die nächste enzymatische Umsetzung verwendet werden kann, eingeleitet. (Schritt c)
Um den Ammoniak aus der Reaktionslösung zu entfernen, wird eine Temperatur zwischen etwa 50 und 100°C eingestellt und bei Normaldruck bis zu einem Druck von 80 bis 120 mbar die Hauptmenge des Ammoniaks ausgetrieben.
Nach etwa 0,5 bis 10 Stunden wird gegebenenfalls bei einem Vakuum von 200 bis 300 mbar Stickstoff durch die Reaktionslösung geblasen und eine Temperatur von 55 bis 65°C gehalten, sodaß auch die Restmenge an Ammoniak entfernt wird.
Die so erhaltene Mono- und/oder Dinatrium-L-Aspartat-Lösung wird sodann im nächsten Schritt d) bei einer Temperatur von 30 bis 60°C mit Benzyloxycarbonylchlorid (Z-Cl) versetzt, wobei durch gleichzeitiges Zutropfen von Natronlauge ein pH-Wert zwischen 9 und 14, bevorzugt zwischen 11 und 12 gehatten wird. Z-CI kann dabei in einer äquimolaren Menge, aber auch in leichtem Überschuß zugesetzt werden. Nach der Z-CI Zugabe wird das Reaktionsgemisch noch 0,5 bis 4 Stunden bei der Reaktionstemperatur stehen gelassen und anschließend auf Raumtemperatur abgekühlt, worauf es zu einer Phasentrennung kommt.

Die wäßrige Phase wird sodann gewaschen, beispielsweise mit Methyl-tert-butylether, Diisobutylether, Toluol oder anderen mit Wasser nicht-mischbaren Lösungsmitteln.
Die organische Phase wird verworfen und Z-L-Asparaginsäure als Dinatriumsalz in wäßriger Lösung erhalten. Zur Entfernung von eventuellen Restmengen an organischen Lösungsmitteln in der wäßrigen Phase, wird bei Raumtemperatur ein Vakuum angelegt.
Durch das erfindungsgemäße Verfahren wird Z-L-Asparaginsäure-Dinatriumsalz als klare, leicht gelbliche Lösung erhalten, die bei Bedarf für die Weiterverarbeitung zu Aspartam, mittels Kopplung mit Phenylalanin verwendet werden kann. Durch das erfindungsgemäße Verfahren werden Ammoniumionen-hältige Abwässer vermieden und Ammoniak quantitativ für weitere enzymatische Umsetzungen von Fumarsäure rückgeführt.
Weiters ist es bei dem erfindungsgemäßen Verfahren nicht erforderlich, L-Asparaginsäure oder eines der Zwischenprodukte zu isolieren.

### Beispiel 1

### a) Enzymatische Umsetzung

In einem 5 l Doppelmantelgefäß wurden 2070 ml Trinkwasser vorgelegt und 973 g Fumarsäure (8,38 Mol) eingerührt. In die Fumarsäuresuspension wurden anschließend 1450 ml 25 Gew.%ige Ammoniak-Lösung (1314 g) während 30 Minuten eingebracht. Dabei wurde eine Reaktionstemperatur von 45 - 50°C und ein pH-Wert von 8,5 erreicht. Bei 45°C und unter leichtem Rühren wurden in die klare Lösung 1,4 ml Aspartase Lösung (245 IU/ml) eingebracht. Danach wurde die Rührung ausgeschaltet und die Reaktionslösung bei 45°C gehalten. Der Reaktionsverlauf wurde photometrisch verfolgt. Eine nahezu quantitative Umsetzung (> 99 %) wurde nach 25,5 Stunden erreicht.

### b) und c) Austreiben von Ammoniak

In die gemäß a) erhaltene Lösung wurde nach Abkühlen auf 25°C 1330 g 50 Gew.%ige Natronlauge innerhalb von 15 Minuten eingebracht. Dabei erwärmte sich die Lösung auf 50°C Es wurde ein pH-Wert von 11,5 gemessen. Der entweichende Ammoniak wurde in eine Fumarsäuresuspension mittels Tauchrohr eingeleitet (Fumarsäure/Ammoniak Waschlösung). Die Wassermenge und Fumarsäuremenge waren dieselben, wie in a) angeführt. Durch Erwärmen auf 57°C und Anlegen eines Vakuums von 100 mbar wurde die Hauptmenge des Ammoniaks ausgetrieben. Nach 6 Stunden wurde Stickstoff durch die Reaktionslösung geblasen (20 I/Std.). Die Temperatur wurde dabei auf 60°C erhöht und ein Vakuum von 240 mbar eingestellt (Dauer 4,5 Stunden). Die Gesamtmenge an Destillat betrug ungefähr 1100 g. Restammoniakgehalt < 100 ppm.

Es wurde eine Dinatrium-L-Aspartat-Lösung mit einem Gehalt von 23,5 Gew.% an L-Asparaginsäure erhalten. Dabei konnte keine Racemisierung beobachtet werden.
Der Gehalt an Fumarsäure betrug < 0,1 Gew.%, Äpfelsäure und Asparagin konnte nicht nachgewiesen werden.

### d) Herstellung von Dinatrium Z-(L)-aspartat

Es wurden 80 ml einer analog Beispiel 1a - c) hergestellten Dinatrium L-Aspartat Lösung mit 18,1 Gew.% an L-Asparaginsäure (0,11 mol) auf 45°C erwärmt. Unter Rühren wurden 20,5 g Benzyloxycarbonylchlorid (Z-Cl) (Gehalt: 92,5 nach GC) (0,11 Mol) während 1 Stunde zugetropft. Gleichzeitig wurde durch Zutropfen von 50 Gew.%ige Natronlauge der pH-Wert zwischen 10,9 und 13,8 gehalten. Insgesamt wurden dabei 7,8 g 50 Gew.%ige Natronlauge zugegeben. Nach dem Zutropfen wurde die Reaktionslösung für 2,5 Stunden bei 45 - 47 °C gehalten. Der pH-Wert betrug konstant 12,1. Nach dem Abkühlen auf 25°C wurde die wässrige Lösung 2 mal mit jeweils 92 ml Methyl-tert- butylether behandelt. Die organische Phase wurde verworfen; die wässrige Phase wurde 30 Minuten bei 20°C und 20 mbar im Rotavapor von Lösungsmittelresten befreit.
Es wurden 101 g einer leicht gelblichen, klaren Lösung erhalten. Der Gehalt betrug 30,8 Gew.% Dinatrium-Z-(L)-Aspartat (berechnet als L-Asparaginsäure)

### Beispiel 2

### Herstellung von Mononatrium-L-Aspartatlösung

Analog Beispiel 1 b) und c) wurde in 1300 ml einer gemäß 1a) erhaltenen Lösung nach Abkühlen auf 25°C 588 g 20 Gew.%ige Natronlauge innerhalb von 15 Minuten eingebracht. Dabei erwärmte sich die Lösung auf 50°. Der entweichende Ammoniak wurde in eine Fumarsäuresuspension mittels Tauchrohr eingeleitet (Fumarsäure/Ammoniak Waschlösung). Die Wassermenge und Fumarsäuremenge waren dieselben, wie in a) angeführt. Bei Normaldruck wurde die Gesamtmenge des Ammoniaks ausgetrieben und die Reaktionslösung auf 1200 ml eingeengt. Restammoniakgehalt < 300 ppm.

Es wurde eine Mononatrium-L-Aspartat-Lösung mit einem Gehalt von 25,9 Gew.% an L-Asparaginsäure erhalten. Dabei konnte keine Racemisierung beobachtet werden.

Der Gehalt an Fumarsäure betrug < 0,1 Gew.%, Äpfelsäure und Asparagin konnte nicht nachgewiesen werden.

Die Lösung wurde analog Bsp. 1d zu Dinatrium Z-(L)-aspartat weiterverarteitet.

### Beispiel 3

### Herstellung von Dinatrium Z-(L)-Aspartat mit einem Überschuß von Z-CI

Es wurden 64,2 ml einer Dinatrium-L-Aspartat Lösung mit 18,1 Gew.% L-Asparaginsäure (0,09 Mol), hergestellt analog Beispiel 1 a - c, auf 45°C erwärmt. Unter Rühren wurden 17,7 g Benzyloxycarbonylchlorid (Z-CI, Gehalt: 92,5 nach GC) (0,10 Mol) während 1 Stunde zugetropft. Gleichzeitig wurde durch Zutropfen von 50 Gew.%ige Natronlauge der pH Wert zwischen 9,4 und 11,8 gehalten. Insgesamt wurden dabei 8,8 g 50 Gew.%ige Natronlauge zugegeben. Nach dem Zutropfen wurde die Reaktionslösung für 2,5 Stunden bei 45°C gehalten. Der pH-Wert betrug konstant 11,3. Nach dem Abkühlen auf 25°C wurde die wässrige Lösung 2 mal mit jeweils 46 ml Methyl-tert-butylether behandelt. Die organische Phase wurde verworfen; die wässrige Phase wurde 30 Minuten bei 20°C und 20 mbar im Rotavapor von Lösungsmittelresten befreit.

Es wurden 84,2 g einer leicht gelblichen, klaren Lösung erhalten. Der Gehalt betrug 31,4 Gew.% Dinatrium-Z-(L)-Aspartat (berechnet als L-Asparaginsäure).

### Beispiel 4:

### Enzymatische Umsetzung der Fumarsäure/Ammoniak-Waschlösung

Die gemäß Beispiel 1 a-c erhaltene Fumarsäure/Ammoniak Waschlösung wurde nach abgeschlossenem Versuch mit 514 g 25 Gew.% Ammoniak auf pH 8,5 gestellt und analog Beispiel 1 a-c zu Dinatrium-L-Aspartat-Lösung weiterverarbeitet.
Die Ergebnisse sind aus Tabelle 1 ersichtlich.

**Tabelle 1**

| Versuch | | 1. | 2. | 3. | 4. | 5. |
|---|---|---|---|---|---|---|
| Wasser | (ml) | 2070 | 2000 | 2423 | 2450 | 2100 |
| Fumarsäure | (g) | 973 | 973 | 973 | 973 | 973 |
| Ammoniak | (g) | 1314 | 514 | 752 | 570 | 643 |
| pH-Wert | bei 45°C | 8,5 | 8,18 | 8,48 | 8,48 | 8,4 |
| Enzym-Lsg | (ml) | 1,4 | 1,4 | 2,1 | 2,1 | 1,4 |
| Dauer | (h) | 25,5 | 24 | 16 | 15 | 28 |
| Umsatz | (%) | >99 | >99 | >99 | 98,6 | >99 |
| 50 % NaOH | (g) | 1330 | 1229 | 1316 | 1333 | 1312 |
| pH-Wert | | 11,5 | 11,9 | 11,6 | 11,9 | 11,8 |
| T max | (°C) | 57 | 51 | 53 | 66 | 59 |
| Vakuum | (mbar) | 100 | 174 | 120 | 200 | 170 |
| Dauer | (h) | 6 | 1 | 2,5 | 3,5 | 2,75 |
| N₂Purge | l/h | 20 | 35 | 30 | 30 | 30 |
| Temp. | (°C) | 61 | 60 | 59 | 70 | 63 |
| Vakuum | (mbar) | 240 | 300 | 250 | 250 | 210 |
| Dauer | (h) | 4,5 | 5 | 3 | 1 | 1 |
| | | | | | | |
| Gehalt | (%L-Asp) | 23,5 | 18,2 | 21,4 | 22,6 | 20,7 |

Die so erhaltenen Dinatrium-L-Aspartat-Lösungen wurden analog Beispiel 1d und Beispiel 3 zu Z-L-Asparaginsäure-Dinatriumsalz umgesetzt.

## Patentansprüche

1. Verfahren zur Herstellung von Z-L-Asparaginsäure-Dinatriumsalz aus Fumarsäure, **dadurch gekennzeichnet, daß**
a) Fumarsäure in einem inerten Verdünnungsmittel in Gegenwart von Aspartase oder Aspartase-produzierenden Mikroorganismen mit Ammoniak zu Ammonium-L-Aspartat umgesetzt, anschließend
b) die Ammonium-L-Aspartat hältige Lösung mit Natriumhydroxid versetzt wird, wobei sich je nach eingesetzter Menge an Natriumhydroxid das Mono- oder Dinatrium-L-Aspartat oder ein Gemisch davon bildet und
c) der sich abspaltende Ammoniak in eine Fumarsäuresuspension, die als Ausgangslösung für weitere enzymatische Umsetzungen verwendet wird, rückgeführt wird, worauf
d) die verbleibende Mono- und/oder Dinatrium-L-Aspartat hältige Lösung mit Benzyloxycarbonylchlorid bei einem pH-Wert zwischen 9 und 14 bei gleichzeitiger Zugabe von Natriumhydroxid reagieren gelassen wird, sodaß sich Z-L-Asparaginsäure-Dinatriumsalz bildet.

2. Verfahren nach Anspruch 1, **dadurch gekennzeichnet, daß** als inertes Verdünnungsmittel Wasser, Wasser/Ethanol oder Wasser/Aceton Gemische verwendet werden.

3. Verfahren nach Anspruch 1, **dadurch gekennzeichnet, daß** Ammoniak verflüssigt oder in Form einer wäßrigen 10 bis 35 Gew.%igen Lösung eingesetzt wird.

4. Verfahren nach Anspruch 1, **dadurch gekennzeichnet, daß** Fumarsäure in Gegenwart von 30 bis 50 IU (Enzymaktivität) pro Mol Fumarsäure umgesetzt wird.

5. Verfahren nach Anspruch 1, **dadurch gekennzeichnet, daß** der sich abspaltende Ammoniak bei Normaldruck bis zu einem Vakuum von 80 bis 120 mbar bei 50 bis 100°C und gegebenenfalls anschließendem Einblasen von Stickstoff bei 200 bis 300 mbar und 55 bis 65°C vollständig aus der Reaktionslösung entfernt wird.

6. Verfahren nach Anspruch 1, **dadurch gekennzeichnet, daß** die Reaktion von Benzyloxycarbonylchlorid mit der Mono- und/oder Dinatrium-L-Aspartat hältigen Lösung bei einem pH-Wert zwischen 11 und 12 durchgeführt wird.

7. Verfahren nach Anspruch 1, **dadurch gekennzeichnet, daß** die Natrium-L-Aspartat hältige Lösung bei 30 bis 60°C mit Benzyloxycarbonylchlorid versetzt wird.

8. Verfahren nach Anspruch 1, **dadurch gekennzeichnet, daß** im Anschluß an die Reaktion von Mono- und/oder Dinatrium-L-Aspartat und Benzyloxycarbonylchlorid, nach erfolgter Phasentrennung, die wäßrige Phase mit einem mit Wasser nicht mischbaren Lösungsmittel gewaschen wird.

9. Verfahren nach Anspruch 8, **dadurch gekennzeichnet, daß** als mit Wasser nicht mischbares Lösungsmittel Methyl-tert-butylether, Diisopropylether oder Toluol verwendet wird.

10. Verfahren nach Anspruch 8, **dadurch gekennzeichnet, daß** die das Z-L-Asparaginsäure-Dinatriumsalz enthaltende wäßrige Phase zur Weiterverarbeitung zu Aspartam mittels Kopplung mit Phenylalanin verwendet wird.

## Claims

1. Process for the preparation of disodium Z-L-aspartate from fumaric acid, **characterized in that**
a) fumaric acid [lacuna] reacted with ammonia in an inert diluent in the presence of aspartase or aspartase-producing microorganisms to give ammonium L-aspartate, then
b) the ammonium L-aspartate-containing solution is treated with sodium hydroxide, forming, depending on the amount of sodium hydroxide used, the mono- or disodium L-aspartate or a mixture thereof, and
c) the eliminated ammonia is returned to a fumaric acid suspension, which is used as starting solution for further enzymatic reactions, and then
d) the residual mono- and/or disodium L-aspartate-containing solution is reacted with benzyloxycarbonyl chloride at a pH between 9 and 14, with the simultaneous addition of sodium hydroxide, to form disodium Z-L-aspartate.

2. Process according to Claim 1, **characterized in that** the inert diluent used is water or water/ethanol or water/acetone mixtures.

3. Process according to Claim 1, **characterized in that** the ammonia used is liquefied or in the form of an aqueous 10 to 35% strength by weight solution.

4. Process according to Claim 1, **characterized in that** fumaric acid is reacted in the presence of 30 to 50 IU (enzyme activity) per mole of fumaric acid.

5. Process according to Claim 1, **characterized in that** all the eliminated ammonia is removed from the reaction solution at a pressure of between atmospheric and 80 to 120 mbar at 50 to 100°C and, if necessary, with subsequent bubbling of nitrogen at 200 to 300 mbar and 55 to 65°C.

6. Process according to Claim 1, **characterized in that** the reaction of benzyloxycarbonyl chloride with the mono- and/or disodium L-aspartate-containing solution is carried out at a pH of between 11 and 12.

7. Process according to Claim 1, **characterized in that** benzyloxycarbonyl chloride is added to the sodium L-aspartate-containing solution at 30 to 60°C.

8. Process according to Claim 1, **characterized in that** subsequent to the reaction of mono- and/or disodium L-aspartate and benzyloxycarbonyl chloride and phase separation, the aqueous phase is washed with a water-immiscible solvent.

9. Process according to Claim 8, **characterized in that** the water-immiscible solvent is methyl tert-butyl ether, diisopropyl ether or toluene.

10. Process according to Claim 8, **characterized in that** the aqueous phase which contains the disodium Z-L-aspartate is used for further conversion into aspartame by coupling with phenylalanine.

## Revendications

1. Procédé de préparation de sel disodique de l'acide Z-L-aspartique à partir d'acide fumarique, **caractérisé en ce que** :
a) on fait réagir de l'acide fumarique dans un diluant inerte, en présence d'aspartase ou de micro-organismes produisant de l'aspartase, avec de l'ammoniac pour obtenir du L-aspartate d'ammonium, ensuite
b) on ajoute de l'hydroxyde de sodium à la solution contenant le L-aspartate d'ammonium, et selon la quantité utilisée d'hydroxyde de sodium, le L-aspartate mono- ou disodique est formé, ou un mélange de ceux-ci, et
c) on recycle l'ammoniac qui se sépare dans une suspension d'acide fumarique qu'on utilise en tant que solution de départ pour d'autres réactions enzymatiques, après quoi
d) on fait réagir la solution restante, qui contient le L-aspartate mono- et/ou disodique, avec du chlorure de benzyloxycarbonyle à une valeur de pH comprise entre 9 et 14, avec addition simultanée d'hydroxyde de sodium, de sorte que le sel disodique de l'acide Z-L-aspartique se forme.

2. Procédé selon la revendication 1, **caractérisé en ce qu'**on utilise, comme diluant inerte, de l'eau ou des mélanges d'eau/éthanol ou d'eau/acétone.

3. Procédé selon la revendication 1, **caractérisé en ce qu'**on utilise de l'ammoniac liquéfié ou sous la forme d'une solution aqueuse à 10 à 35 % en poids.

4. Procédé selon la revendication 1, **caractérisé en ce qu'**on fait réagir de l'acide fumarique en présence de 30 à 50 UI (activité enzymatique) par mole d'acide fumarique.

5. Procédé selon la revendication 1, **caractérisé en ce que** l'ammoniac qui se sépare est complètement séparé de la solution de réaction, à une pression comprise entre la pression normale et un vide de 80 à 120 mbar, à une température de 50 à 100°C, ceci étant suivi, le cas échéant, d'une insufflation d'azote à 200 à 300 mbar et à 55 à 65°C.

6. Procédé selon la revendication 1, **caractérisé en ce qu'**on effectue la réaction du chlorure de benzyloxycarbonyle avec la solution contenant le L-aspartate mono- et/ou disodique à une valeur de pH comprise entre 11 et 12.

7. Procédé selon la revendication 1, **caractérisé en ce qu'**on ajoute du chlorure de benzyloxycarbonyle à la solution contenant le L-aspartate sodique à une température de 30 à 60°C.

8. Procédé selon la revendication 1, **caractérisé en ce que**, directement après la réaction du L-aspartate mono- et/ou disodique et du chlorure de benzyloxycarbonyle, quand la séparation des phases est terminée, on lave la phase aqueuse avec un solvant non miscible avec l'eau.

9. Procédé selon la revendication 8, **caractérisé en ce qu'**on utilise, comme solvant non miscible avec l'eau, de l'éther de méthyle et de tertio-butyle, de l'éther diiso-propylique ou du toluène.

10. Procédé selon la revendication 8, **caractérisé en ce qu'**on utilise la phase aqueuse contenant le sel disodique de l'acide Z-L-aspartique pour une transformation supplémentaire en aspartame au moyen d'un couplage avec de la phénylalanine.
